# EUROPEAN PATENT APPLICATION

(11) **EP 3 845 691 A1**
(43) Date of publication of application: **07.07.2021**
(21) Application number: 20150111.1
(22) Date of filing: 02.01.2020
(51) Int. Cl.: D01G 11/00, A61L 2/10, D06M 10/00

(54) **METHOD AND APPARATUS USING UV-A AND UV-C TO TREAT TEXTILE MATERIALS AND METHOD AND SYSTEM OF PROCESSING WASTE FABRICS TO REJUVENATED FIBROUS MATERIALS**

(71) Applicant: Nunn, Kayren Joy, Arlington TX 76017 (US)
(72) Inventor: Nunn, Kayren Joy, Arlington TX 76017 (US)
(74) Representative: Lecomte & Partners

(57) **Abstract**

A method of processing waste fabrics to rejuvenated fibrous materials includes obtaining fabric data for each bale of incoming waste fabrics and storing the fabric data in a database; obtaining target data relating to target product requirements; processing the fabric data stored in the database and the target data according to predetermined algorithms to generate a rejuvenation processing recipe which specifies bales information relating to bales of incoming waste fabrics selected for further rejuvenation processing, and rejuvenation processes information relating to a series of processes and corresponding process parameters for each of the series of processes for processing the selected bales of incoming waste fabrics to obtain rejuvenated fibrous materials specific to the target product requirements; Selecting, according to the bales information of the rejuvenation processing recipe, corresponding bales of incoming waste fabrics for further rejuvenation processing; and subjecting the selected bales of incoming waste fabrics to processes specified by the rejuvenation processes information of the rejuvenation processing recipe to obtain rejuvenated fibrous materials specific to the target product requirements.

## Description

### Technical Field

The present invention is generally in the field of equipment and methods for exposing textiles to, and/or treating textile materials with, ultraviolet light, more particularly, with a combination of UV-A and UV-C light. The present invention generally relates to the recycling of post-industrial and pre-consumer materials. In particular, the present invention relates to a method and corresponding system of processing waste fabrics to rejuvenated fibrous materials that will maintain their original quality in downstream production without a loss of downstream manufacturing efficiencies. This allows for a higher quality product that is more sustainable and utilizes hundreds of billions of pounds of waste that would typically be landfilled, incinerated, or downcycled annually.

### Background of the Invention

The recycling industry has historically considered all textile waste material to be peripheral, relegating it to be swept from cutting tables and mill floors and deposited in bins or baled along with any foreign debris mingled with it. If the scrap were recycled, all of the now indigenous debris would be recycled with it, and therefore included in down-cycled products where quality criteria are not strictly adhered to.

About half of the world's wastewater problems are linked to producing textile goods, and many of the chemicals used to dye and finish fabrics are known to harm human health. Often, clippings from carpet or fabric mills are replete with dangerous chemicals and are handled like toxic waste. Ironically, many of the products made from these materials are considered safe for use in the home.

Since the beginning of the 21st century, environmental concerns have become central to the global populace and designers in many fields have begun to reconsider their role in the production and consumption of consumer goods. The design community has concluded that without using sustainable fibers which are equal in quality and price to those of virgin fibers, the global impact will remain negligible without exception.

As designers embrace new technologies and materials, they must address the ecological impact of their designs in order to shape a more sustainable future. The 21st century has marked the beginning of a new textile revolution, as it is becoming intelligent, sustainable and ethical to find solutions to this global waste crisis. Methods for producing high-quality rejuvenated textile waste fibers are dependent upon critical data as well as specialty equipment and technologies.

The amalgamation of this approach becomes evident in establishing a rejuvenation process based upon both intuitive and scientific data analysis. Furthermore, quality control systems have been instituted which are singular within the industry, as well as unique chemistry and engineered equipment which gently, not aggressively, deconstruct fabrics and revert them to yarn segments (Pre-Fiberization) and ultimately to individual fibers (Fiber Refinement).

Fiber Rejuvenation 1s a holistic system and, if approached in this manner, can transform what is now a "niche" down-cycled market for apparel and industrial textiles into a mainstream sustainable raw material that is equal in quality, and costs less, than virgin raw material. As such, rejuvenated fibers can create value to the industry and its consumers.

It would be advantageous to have processes for segregating families of fibers and purifying those fibers so that they can be competitive with their virgin counterparts, in a cost- competitive and environmentally sustainable manner.

Before post-consumer and/or post-industrial textile waste is converted to fibers, it also can be advantageous to separate fabrics made from natural fibers from fabrics made from synthetic fibers, and/or to separate and/or segregate fabrics which have been treated with optical brighteners from those which have not been treated with optical brighteners. In this manner, the segregated and/or separated fabrics and textile materials can be treated differently, using treatments appropriate for the types of fibers and the types of finishes, before mechanically breaking the fabrics down into fibers. It can also be advantageous to disinfect the textile waste, as it may be taken from landfills and other locations where it is less than sanitary.

The present invention provides such processes.

### Brief Description of the Drawings

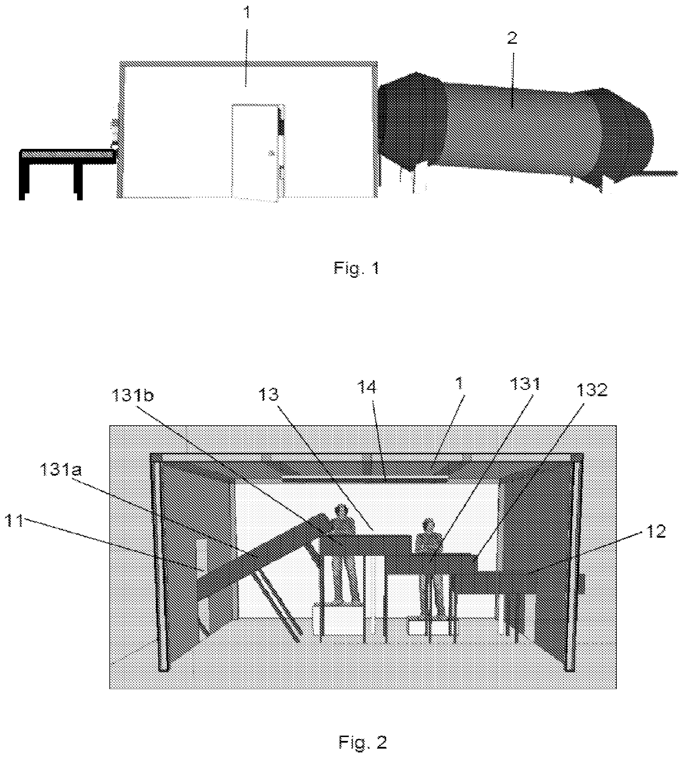

Fig. 1 illustrates the structure of the apparatus of an embodiment of the present invention.
Fig. 2 illustrates the structure of the segregation chamber of the apparatus of an embodiment of the present invention.

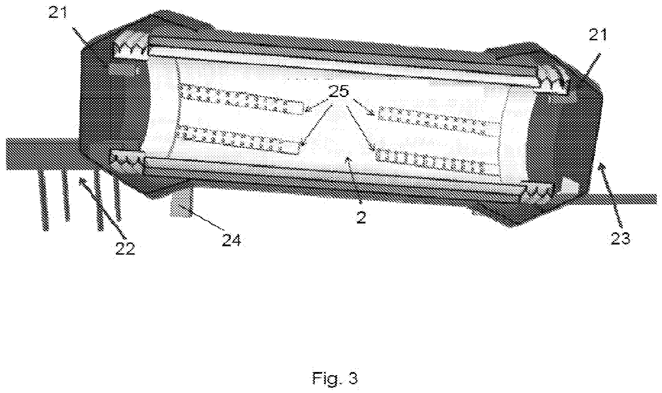

Fig. 3 illustrates the structure of the sterilization chamber of the apparatus of an embodiment of the present invention.
Fig. 4 is a chart showing the effectiveness of UV exposure as a disinfecting treatment.
Figure 5 is a top view of a representative UV chamber that can be used to treat the fibers/non-woven materials described herein, showing how a conveyor belt traversing a UV chamber.
Figure 6 is a front view of representative UV chamber, showing a conveyor belt traversing the chamber, under a series of UV lamps.
Figure 7 is a side view of representative UV chamber, showing a conveyor belt traversing the chamber, under a series of UV lamps.

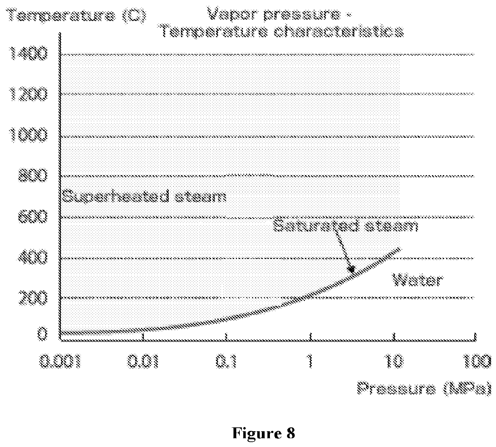

Figure 8 is a chart showing the vapor pressure/temperature characteristics of water, saturated steam, and superheated steam, in terms of temperature (°C) and pressure (mPa).

### Representative Embodiment

In this representative embodiment, the apparatus comprises a segregation chamber 1 and a sterilization chamber 2. In one embodiment, the segregation chamber 1 has an input end 11 and an output end 12 which receives incoming textile materials from the input end 11 and separates optically brightened textile materials and/or synthetic fiber materials from the incoming textile materials. A cascading conveying means 13 and a UV-A radiation source 14 are disposed within the segregation chamber 1. The cascading conveying means 13 traverses the incoming textile materials from the input end 11 to the output end 12 of the segregation chamber 1 and operates to flip and turn the incoming textile materials with minimal human contact. The cascading conveying means 13 defines a manual separating station whereat detected optically brightened textile materials and/or synthetic fiber materials are removed from the cascading conveying means 13. The cascading conveying means 13 comprises a plurality of conveyor belts 131 arranged in a cascade manner, wherein each of the conveyor belts 131 has an outlet end 132, and the conveyor belt 131 lower down projects forwardly of the outlet end 132 of the conveyor belt 131 thereabove. The conveyor belt 131 may for example be 6 foot long by 3 foot wide, but it could be shorter or longer depending on the production rate needed of the production line. The plurality of conveyor belts 131 comprises a first conveyor belt 131a which connects the input end 11 of the segregation chamber 1 to a subsequent conveyor belt 13 Ib, and the first conveyor belt 13 Ia is slantedly disposed so that the incoming textile materials traverse upward from the input end 11 of the segregation chamber 1, which is at a lower position, to the subsequent conveyor belt 13 lb, which is at an upper position.

The UV-A radiation source 14 irradiates the cascading conveying means 13 with UV- A radiation for detection of optically brightened textile materials and/or synthetic fiber materials. It is disposed on top of the cascading conveying means 13 and delivers UV-A radiation with a wavelength between 3 nanometers and 400 nanometers. In other embodiment, an optically brightened textile materials and/or synthetic fiber materials separating means may be disposed within the segregation chamber 1 and coupled to an area external to the segregation chamber 1, whereby detected optically brightened textile materials and/or synthetic fiber materials are removed from the segregation chamber 1. The optically brightened textile materials and/or synthetic fiber materials separating means is a vacuum area wherein the detected optically brightened textile materials and/or synthetic fiber materials are transported to the area external to the segregation chamber 1 by negative pressure. The detected optically brightened textile materials and/or synthetic fiber materials are manually removed from the segregation chamber 1.

In this embodiment, the segregation chamber 1 is also disposed with air ventilation and filtration means (not shown) to remove fine dust particles from the air in the segregation chamber. The sterilization chamber 2 receives textile materials output from the segregation chamber 1, and is disposed with a UV-C radiation source 21 therewithin. The UV-C radiation source 21 irradiates the sterilization chamber 2 with UV-C radiation for sterilization and surface modification of the textile materials to increase wettability, absorbability and reduce pilling. The UV-C radiation source 21 delivers UV-C radiation with a wavelength between 0 nanometers and 280 nanometers, and is a pulsating UV-C light source.

The sterilization chamber 2 is in form of a rotating cylinder having an input end 22 and an output end 23, wherein the textile materials traverse from the input end 22 to the output end 23 as they are irradiated by the UV-C radiation source 21. The rotating cylinder is approximately 1-2 meters in diameter and 3- meters long, and it is mounted on a stand 24 with an effective slope of -0.12 to -0.16. It has an interior surface which is highly reflective. The interior surface of the rotating cylinder is disposed with a plurality of rows of opening slats which are angled with a travel axis along which the fabric pieces travel in the rotating cylinder, and the rows are offset from each other. The opening slats are made of highly reflective material, and they assist in the opening or "unfolding" of the textile materials while the materials are being tumbled in the rotating cylinder.

In addition to using a segregation station to provide a source of UV-A light to identify materials which include optical brighteners, or which include synthetic fibers, and remove them from textile materials including natural fibers, which have not been treated with optical brighteners, and a disinfection station using UV-C to disinfect/sterilize the materials and/or fibers, optional additional components can be present. These components can deconstruct/fiberize the material, can steam treat the material, and can apply chemical treatments to the material (referred to herein as "catalyzed vapor."

For the purpose of this illustration, and many other scenarios encompassing a broad spectrum of fabric deconstruction, individual treatment and deconstruction groups can be used in the following manner.

In the first treatment station, a softener can be applied and surface chemicals present on the textile material to be treated can be removed from the textile materials in a cleansing stage.

For example, in the case of cotton, these surface chemicals often include starches, waxes, anti-crease finishes, hydrophobic finishes, and the like. In the case of synthetics, the finishes often include polycarboxylic acids (PCA), acrylic sizing agents, oiling waxes or silicone based lubricants, and combinations thereof.

The softener used in the instant process can act to soften the surface of the fabric in a controlled environment, in order to relax it so that the deconstruction process can be more effective and less invasive.

In a subsequent treatment station, a further chemical can be used to strengthen the yarn segments as they are being deconstructed. This can help maintain the integrity of the fibers therein. Para-KRC from the Kunal Group is an excellent product that can be used to increase tear and tensile strength for silk.

Multiple surfactants can be used for cotton, and a product similar to Avco-Soft PE is excellent when catalyzed for PVA in this area of the process.

A product using a poly (vinylamine-vinylformamide) copolymer with a carrier can be used to improve the strength of fibers that have been weakened, for example, as a result of mechanical stress in the case of a natural hair collection such as cashmere.

These strengthening treatments can be used to increase the tensile strength of the treated fibers, relative to untreated fibers.

In one embodiment, a further treatment includes the application of an antimicrobial agent, which is particularly useful if the downstream product is going to be used in the healthcare, medical, personal care or baby products industries. While it is not necessarily the final process in the system, it is a strategic stage to pretreat for microbial protocols.

If in a downstream application it is necessary for a particular fiber to have a hydrophobic property, a pre-treatment can be applied in a treatment station. If in a downstream application it is important for a particular fiber to have a more hydrophilic nature, a pre-treatment can be applied in a treatment station.

Due to the nature of this technology and its importance in high quality downstream applications, there are many catalytic vapors (treatment chemicals) that can be delivered to improve the performance of the end use fibers during this stage of the process.

In the deconstruction station of each treatment and deconstruction group, there are two rotary drums which are cooled as described above and are populated with sleeves of industrial cutting needles designed specifically for the deconstruction of this fabric into yarn segments. The rotary drums vary in size based on the width of the line, and the width of the line determines the production capacities.

In a representative embodiment of a line producing approximately 2,500 pounds per hour, in one aspect of this embodiment, the rotary drums are approximately 500 mm in diameter and approximately 2,500 mm wide. Each such rotary drum can hold from 20,000 to 120,000 industrial cutting needles, which deconstructs the textile materials passing through the rotary drums as they are prepared for further fiber refinement.

As the yarn segments pass through the deconstruction stations, the cutting needles are designed so that their function is very specific, taking the textile material through each stage of the process.

For example, in the initial stage prior to surface cleansing, the textile material can be penetrated by the cutting needles in preparation for its cleansing by enzymes and chemicals. The textile material can then be treated, for example, with softening agents. After the textile material has been softened and the yarn segments begin to be exposed, there is still the visible presence of textile material that must be penetrated and separated into yarn.

A further deconstruction station is typically the section where the cutting needles begin to separate the yarn segments which yields no trace of actual textile material present, only yarn segments.

By the time the segments reach the fourth or subsequent deconstruction station (dependent, of course, on the type of fabrics being rejuvenated), the yarn segments will have been properly softened and strengthened, prepared for downstream processing, and ready to be delivered to the output storage box prior to subsequent fiber refinement processing.

The apparatus can be used in treatment and deconstruction methods which convert discarded textile materials into fibers that compete or surpass the quality in the marketplace with that of their virgin counterparts.

If desired, during each stage, individual soft yarns can be moved out of the apparatus, where they can be stored, or sent on to receive further refinement. Heavier fabric pieces can be moved forward through the mechanical process until they have been deconstructed into soft yarn elements. If there are elements of textile material that are not properly separated into yarn segments during each stage of the process, they can be redeposited to an earlier stage in the process.

If sufficient yarn separation has not occurred, for example, by the fourth stage of the process, the elements of the textile material can be re-deposited into the second treatment and deconstruction group. Should the separation be inadequate in the third stage of the process, the elements of textile material can be re-deposited into the initial feedbox so as to prevent any unnecessary fiber breakage after the fabric has been deconstructed. The mechanical process to untwist the fibers can become more aggressive at this stage, so it can be important to strengthen the fibers so there is no degradation.

While not wishing to be bound to a particular theory, it is believed that the success of this technology depends on the maintenance of the length and the strength of the fibers at each stage of the process, and this is ultimately achieved by having a Stage Gate Process which revolves around mechanical, chemical and vapor techniques.

To emphasize, when working with cotton or other natural fibers, the strength of the fibers can be increased by adding warm, moist treatments, which penetrate the fabric and
ensure that it is not being weakened, and that additional breakage is not created during the process.

The present invention will be better understood with reference to the following non- limiting examples.

### Example 1: Exposure of Non-Woven Webs to UV Light

As a proxy for the UV-C treatment of textile materials, a series of non-woven webs with a density of 27 g/ft2 were evaluated in a UV light system, in an effort to identify suitable decontamination conditions. The webs were contaminated with a variety of microbes, including E. coli, Staph. aureus, Pseudomonas aeruginosa, Candida albicans, Salmonella enteritidis, Bacillus subtilis, and Aspergillus niger spores.

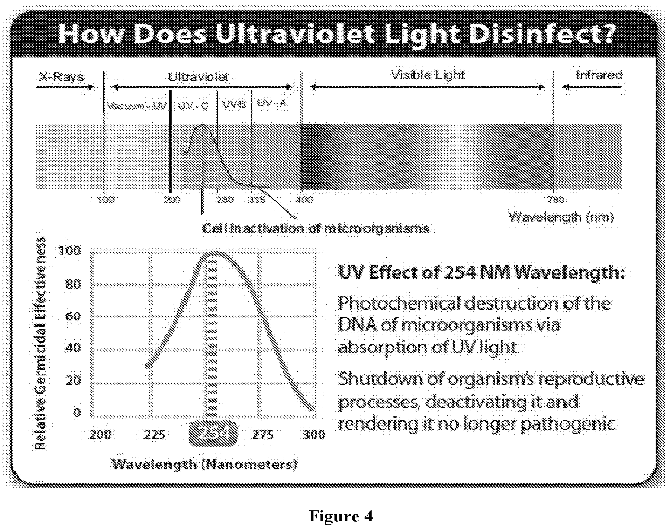

As shown in Figure 4, UV light, particularly light in the UV-C range, is known to have antimicrobial properties. However, it is also known to damage textiles and fibers when the intensity of the light is too high.

In an effort to obtain an antimicrobial effect without damaging the non-woven materials that were being evaluated, the UV lights were set a certain distance, namely, one foot, from the materials. However, satisfactory results are generally obtained when the distance is between about 6 inches to about 2 feet from the non-woven materials, or fibers, to be treated.

A medium intensity UV-C lamp (12 lamp 540W output) system was used, and the exposure time was 3.7 seconds, although a more typical range of exposure times is around I to around 45 seconds, more typically between around 4 and around 7 seconds. A mirrored backing was used to bounce back the light onto the substrate, in order to enhance the effect, though such is not necessary.

The results are tabulated below.

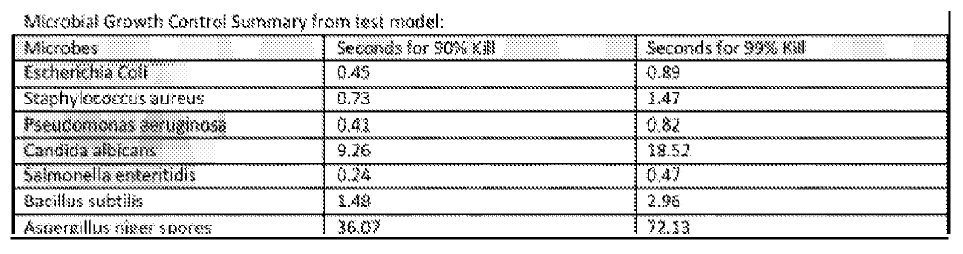

In summary, the UV treatment is extremely effective at disinfecting, and in sterilizing, non-woven materials.

### Example 2: Representative UV Sterilization Chamber

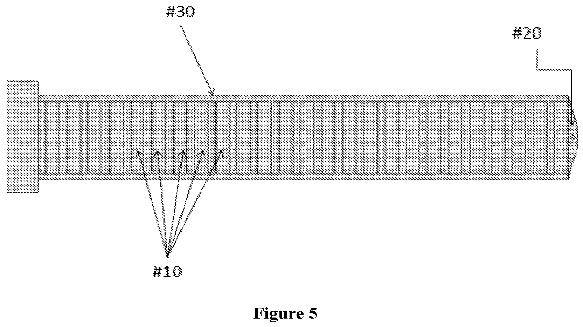

As illustrated in the accompanying drawings, the present invention also provides an apparatus for implementing the aforementioned method utilizing UV-C for treating the textile materials. A representative UV-C treatment module (also referred to as a UV sterilization chamber, or sterilization chamber) is shown in Figures 5-7.

Figure 5 shows a top view of a UV-C treatment module. A conveyor belt (not shown) moves materials through the module. UV lights (10) are present throughout the module, and a suction hood (20) is located at the end of the module where the materials exit the module. The UV lights are housed in an enclosure (30).

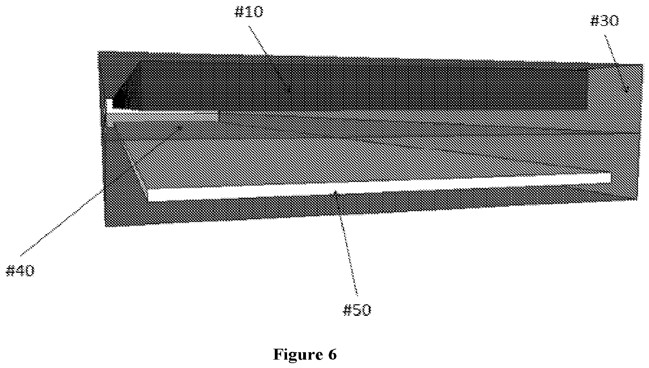

Figure 6 shows a front view of a UV-C treatment module. A conveyor belt (50) passes under UV lights (10) located in an enclosure (30). In this embodiment, a transparent protective layer (40) is present over the lights to prevent contamination of the materials in the event one or more of the UV bulbs breaks.

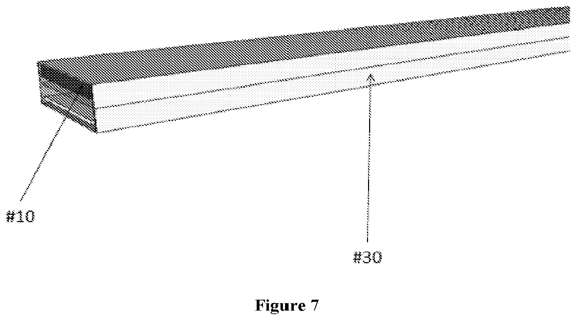

Figure 7 shows a side view of a UV-C treatment module. In this figure, the conveyor belt is not shown. The enclosure (30) houses a series of UV lights (10) at the top, and the textile materials pass under the UV lights (10).

The sterilization chamber receives textile materials, and is disposed with a UV-C radiation source therewithin. The UV-C radiation source irradiates the sterilization chamber with UV-C radiation, which disinfects, and, optionally, sterilizes the fibers and/or textile materials. In some embodiments, it can also modify the surface of the fibers and/or non- woven textile materials, for example, to increase wettability, absorbability and reduce pilling.

The UV-C radiation source delivers UV-C radiation with a wavelength between 100 nanometers and 280 nanometers, and can be a pulsating or constant UV-C light source.

The sterilization chamber described in this example is different from the sterilization chamber described in the above description, which is in the form of a rotating cylinder having an input end and an output end, wherein the fibers and/or textile materials traverse from the input end to the output end as they are irradiated by the UV-C radiation source. The rotating cylinder can be approximately 1-2 meters in diameter and 3-5 meters long, and can be mounted on a stand with an effective slope of -0.12 to -0.16.

As with the earlier-defined rotating cylinder, the chamber in this example can have an interior surface which is highly reflective.

It is an object of the present invention to provide a rejuvenation method and system that create a cost savings to both the downstream manufacturer and consumer, along with a positive CO2 condition, which offer a technological breakthrough for the environmental community.

It is another object of the present invention to use quality materials at their peak without any loss of efficiency in downstream manufacturing, resulting in the value of the fibers increasing significantly over those of "shoddy" fiber production, which makes this a viable and sustainable business opportunity with benefits not only for the environment, but also for industrial partnerships and consumers.

The method and process for producing high quality rejuvenated textile waste fibers is dependent upon critical data as well as specialty equipment and technologies. This amalgamation becomes evident in the establishment of a rejuvenation process based upon both intuitive and scientific analysis of data and proper science of manufacturing. Furthermore, quality control systems have been instituted which are singular within the industry, as well as unique chemistry and engineered equipment which gently, not aggressively, deconstruct fabrics, reverting them to yarn segments (Pre-Fiberization) and ultimately to individual fibers (Fiber Refinement). Fiber Rejuvenation is a holistic system and, if approached in this manner, can transform what is now a "niche" downcycled market for apparel and industrial textiles into a mainstream sustainable raw material that is equal in quality and costs less than virgin raw material and creates value to the industry and its consumers.

To attain this, the present invention provides a method of processing waste fabrics to rejuvenated fibrous materials, comprising the steps of:
Obtaining fabric data for each bale of incoming waste fabrics and storing the fabric data in a database;
obtaining target data relating to target product requirements;
Processing the fabric data stored in the database and the target data according to predetermined algorithms to generate a rejuvenation processing recipe which specifies bales information relating to bales of incoming waste fabrics selected for further rejuvenation processing, and
rejuvenation processes information relating to a series of processes and corresponding process parameters for each of the series of processes for processing the selected bales of incoming waste fabrics to obtain rejuvenated fibrous materials specific to the target product requirements;
Selecting, according to the bales information of the rejuvenation-
processing recipe, corresponding bales of incoming waste fabrics for further rejuvenation processing;
Subjecting the selected bales of incoming waste fabrics to processes specified by the rejuvenation processes information of the rejuvenation-processing recipe to obtain rejuvenated fibrous materials specific
   to the target product requirements.

The present invention also provides a system of method of processing waste fabrics to rejuvenated fibrous materials, comprising:
(a) means for obtaining fabric data for each bale of incoming waste fabrics;
(b) means for obtaining target data relating to target product requirements;
(c) means for processing the fabric data stored in the database and the target data to generate a rejuvenation processing recipe which specifies bales information relating to bales of incoming waste fabrics selected for further rejuvenation processing, and rejuvenation processes information relating to a series of processes and corresponding process parameters for each of the series of processes for processing the selected bales of incoming waste fabrics to obtain rejuvenated fibrous materials specific to the target product requirements.

### Best Mode for Carrying out the Invention

Each of the steps in the method of the present invention is further described below in further details. Step (a):
The first step is to obtain an exact knowledge of materials content of the incoming waste fabrics. In conventional recycling centers, the raw material source is known only when it arrives at the facility and the origin and details of the material, other than fundamental data, is never deemed necessary since its end use is destined for a lower-end commodity. For example, a recycling center may know that the material it will be processing is denim to be used for automotive shoddy pads, but they do not know from where the denim originated nor do they do know the type of finish it has. Moreover, they do not know if the denim is a blend of cotton/polyester, cotton/rayon, or cotton and lycra because that data is not important to the successful manufacture of their product. Conversely, if that denim was going to be woven into a high-end yarn application in a large textile mill where speed and efficiencies were important, those details would be of prime importance. If polyester was present in the blend that was about to go through the rejuvenation processes as in Step (c) of the method of the present invention, there would be streaking in the ultimate yarn. If there were Lycra fibers, the spinning frames would shut down operation due to a problem that no one could pinpoint. Similarly, if a downstream product is calling for white 100% cotton, non-optic material, it is important to understand the type of bleaching and finishes that have gone on the fabric prior to the material being brought into the facility and staged for the rejuvenation processes.

To attain the above object, the fabric data in Step (a) includes composition of the incoming waste fabric, approximate yarn count of the incoming waste fabric, types of treatments or finishes that the incoming waste fabric have received during production of the incoming waste fabric, and color and shade of the incoming waste fabric. The fabric data could be obtained by conducting laboratory tests to the incoming waste fabrics; the laboratory tests may include but are not limited to one or more of the following: burn test, solubility test, dye staining test, Fourier Transform Infrared spectroscopy, chemical reaction test, burn rate test, elemental analysis, color and shade test, UV spectroscopy imaging and detection. It might also include moisture absorption testing for natural or synthetic fibers. The fabric data will determine what chemicals remove the fabric finishes and prepare them for quality fiberization, the types of needles and needle patterns that will be used to initiate the separation of yarns and the untwisting of the threads and so forth. The fabric data also enables the process to individually manage and control the distinct characteristics of the production of fibers which are colored, natural, bleached white and non-optically brightened, thus resulting in the ability to produce quality dyed fiber without using additional dyes or chemicals adds to the sustainability of their integration in all downstream consumer products, and the ability to segregate quality non-optically brightened fiber from one which has been subjected to chlorine bleaching which is important to many consumers. All of this information is considered critical data and therefore key to the ultimate success of the present invention. The fabric data is all entered and stored into a database of an internal intelligence system, RDS (Rejuvenation Data System) and used to set the intelligence processes in motion.

However, it is recognized that it is more cost effective if fabric composition has been predetermined upstream prior to reaching the fiber rejuvenation facility. In that case, the incoming waste fabrics are most effective if they are post-industrial waste fabrics which are sorted preliminarily by fabric color and fabric type but certainly could be post consumer materials, with the importance being that testing would need to occur to determine their composition and finishes once they arrived into the manufacturing facility. In the case of post industrial a lot of knowledge can be obtained directly from the supplier of the incoming waste fabrics but still remains to be verified at the rejuvenation facility for quality purposes.

After obtaining the fabric data and storing the fabric data in the database, the bales of incoming waste fabrics are sorted into batches according to the obtained fabric data, so that appropriate lots may be run simultaneously to ensure greater consistency and quality in their processing. Each sorted batch or bale may weigh from approximately 100 lbs. to 2000 lbs.

Step (b): Target data relating to target product requirements are obtained from users. Target data may include fabric composition (e.g. 60% cotton and 40% polyester), yarn count, color and shade and so forth. It may also include the types of chemicals and/or fabric finishes that are to be included or excluded; this would be of immense importance for specific applications such as sensitive health or baby care or a downstream yarn/fabric target.

Step (c): After the target data are obtained from users, the fabric data stored in the database of the intelligence system is processed according to predetermined algorithms to generate a rejuvenation processing recipe which specifies bales information relating to bales of incoming waste fabrics selected for further rejuvenation processing, and rejuvenation processes information relating to a series of processes and corresponding process parameters for each of the series of processes for processing the selected bales of incoming waste fabrics to obtain rejuvenated fibrous materials specific to the target product requirements. It is important to note that as the present invention captures all relevant fabric data of the incoming waste fabrics at the start, it is possible to make maximum use of the incoming waste fabrics. For example, if
the target product requires for marl or stock dyed yarns, the present invention may select incoming waste fabrics which have been dyed for further rejuvenation processes so that it would not be necessary to incur additional costs or chemicals for dyeing; if the target product requires white cotton for medical or personal care use, the present invention may select incoming waste fabrics of white color which do not have optical brightening agents applied.

Step (d): After the rejuvenation processing recipe has been determined, the next step is to select, according to the bales information of the rejuvenation processing recipe, corresponding bales of incoming waste fabrics for further rejuvenation processing. As the fabric data of all incoming waste fabrics are stored in the database, the current process involves identifying the bales of incoming waste fabrics with fabric data matching to the bales information, and updating the database after the matching bales of incoming waste fabrics are selected and removed for further rejuvenation processing; such database processing techniques should be obvious in the prior art and so no further details are provided herein.

Step (e): The selected bales of incoming waste fabrics will then be subjected to processes specified by the rejuvenation processes information of the rejuvenation-processing recipe. The following describes some of the possible processes in detail: First, opening and simultaneously blending the selected bales of incoming waste fabrics to obtain initially opened and blended fabric pieces. The initially opened and blended fabric pieces are reduced to an appropriate size for conforming to downstream production equipment, and thereafter blended to avoid segregation and to unify composition of the fabric pieces. The size reduction is ideally accomplished using a guillotine-style cutter, although other styles of cutters may perform this function. The goal is for the material to be approximately six to fourteen inches long or six to fourteen inches square at this stage in the process. Once the materials have passed through the size reduction area and become conformed to downstream production equipment, it is important relative to the quality requirements of the method that they are again blended to avoid segregation of color or material type, and to unify the compositions of the fabrics.

Then, cleaning the initially opened and blended fabric pieces to obtain cleaned fabric pieces. In the upstream preparation, residua such as pattern markers or paper will sometimes be found in the folds of the material. As the size of the material begins to be regulated, strings are formed that need to be removed before further processing. Likewise, there is always residual dust, which has been left inside the bales of material that must be removed. These are all lightweight materials that require extraction from the fabric before further processing. The net effect of this procedure is to clean the raw materials from paper, threads and dust which have entered the facility from outside sources, or have been produced in the initial size reduction of the raw materials.

To further control quality, UV light is added to the process to detect foreign chemicals such as optical brighteners and traces of synthetics or color contaminants when they are either undesirable in downstream production or when the application requires a 100% organic product.

The fabric pieces then pass through a subsequent stage to further reduce their size, as it has been important to maintain a relatively large size up to this point in the process. It is now necessary to reduce the fabric pieces to a uniform size of approximately three to six inches square or, if they are rectangular in shape, a maximum length of six inches is considered optimal, based on the type of fiber that will begin to be refined back to a virgin state. This is best performed by a second guillotine cutter, but can be accomplished by any other type cutter providing the blades are kept sharp to keep dust and threads to a minimum during the process.

Once the fabric pieces have been reduced in size, they will be conveyed to the initial Fabric Deconstruction Group for its surfaces to be penetrated by sleeves of industrial needles, which prepares the fabric pieces for an organic bio surface-cleaning step in the process. At this point the materials will be pneumatically suctioned to an intimate blending area and centralized production storage bin.

As the process progresses, a continuous process batch are created and a critical element is initiated, namely, the blending of a complete batch of fabrics which results in the creation of a more harmonious blend of rejuvenated fibers. This is accomplished when a delivery condenser carrying fabric pieces positions itself over a large blending box, which is approximately 10 feet wide and 40 feet long. The delivery condenser moves from right to left over the box dropping the material onto its floor in a z-type pattern until the floor has been covered, and repeats the process until the box has been filled.

The blended fabric pieces are then subjected to cleaning process to remove fabric finishes. Utilization of a semi-dry method of removing these finishes is preferred wherein specific enzymes targeting specific finishes are applied to the fabric pieces in form of vapor and/or spray in such a way that moisture content in the fabric pieces at this stage would not exceed 20%, and as the fabric pieces pass through the rest of the rejuvenation processes, the end result of moisture never exceeds 15% throughout the overall manufacturing process. It is important to target only what is prescribed for removal, thus maintaining the integrity of the materials in the process. Broad-spectrum enzymes used without knowledge of what is specifically on the fabric become nothing more than a guessing game. The utilization of the fabric data which is obtained at the outset of the present invention is therefore critical in making assessments for quality downstream products used in high value applications, especially in the health, baby and personal care marketplace.

By using the semi-dry method, the moisture from the vapor and the chemistry is important to maintain the length and the strength of the fibers that are being processed throughout the system. In rejuvenating cotton fabrics into fiber, it is always important to remove these starches, silicones or other unique finishes which were placed on the fabric during its original manufacture process. Specialized enzymes perform well in this endeavor. In the case of rejuvenating wool fabrics, enzymes would never be used at this stage since they attack the cell membrane of the complex wool fibers and lead to the disintegration of the structure into its component cortical cells. This would lead to holes in the fabric and degradation of the ultimate fiber. Therefore, each particular fiber group is dealt with individually based on the fabric data fed into the RDS internal intelligence system.

The initial cleaning of the fabric is important due to a number of factors relative to both the pre-fiberization process as well as its eventual downstream application. (1) It is important to remove as much of the surface finish as possible to prepare the materials for the deconstruction and pre-fiberization process. This is considered an important part of the purification process. This enables the chemical surface bonds from the fabric finishing, which has occurred upstream to be cleaned from the fabric, and allows for a gentle, non- invasive deconstructive process to begin. (2) In some instances, it is important once the surface bond is broken and cleaning has taken place, that moisture be added as a component into the fiber to strengthen it downstream, (a) In the case of cotton, moisture is important to its preparation since dehydration renders cotton fiber weak and brittle causing it to break and become shorter. If a cotton fiber is made shorter in this process, it will not process efficiently in downstream applications. (b)The compound chemistry introduced in the process adds a strengthening agent to the fiber, thus enhancing its performance through fiberization and downstream applications. (3) If fabric finishes have not been eradicated during the cleaning technique in the fiberization process, the probability exists that residual finishes will promote streaking or barring when the fabric is dyed, which is unacceptable in the manufacture of quality knits, woven' s or non-woven for the marketplace. (4) Fabric cleaning advances a gentler fiberization process resulting in minimal dust in the ambient air and less short fiber content in the actual product.

Once the fabric pieces are cleaned, a spiked apron will retrieve the cleaned fabric pieces from the end of the box and pneumatically deliver it using a negative pressure system into a vertical transfer unit so that a cross section of the material will be a harmonious blend of material delivered to the next process stage of fabric deconstruction and pre-fiberization.

The materials are now air conveyed by suction to an inline feed box to prepare for the deconstruction/pre-fiberization stage of the process. It is important to note that in preferred embodiments fans are never used to blow the material from place to place, which creates knotting ("neps") in fiber and results in a lower quality end product. Only the use of a negative pressure transport system, in this case suction and/or vacuum, is considered an acceptable conveyance with regard to moving material from one process to another.

Some fabrics are loosely constructed, thus a novice in the art of textiles would understand that a "cable knit" sweater would be less structured in its weave than a pair of jeans, but not as fine a weave as a man's dress shirt or a ladies' silk dress. The present invention's use of information regarding yarn count and weave/knit or non-woven structure is an important technological part of the process, which enables it to be accurate in creating the highest quality fiber possible.

To understand the distinct differences between rejuvenation technology and that of classic recycling, it is important to note the difference in the structure of textiles and the data that is crucial to the information systems in order to finalize decisions on which types of equipment will be used to optimize the process and create the best quality fiber for any desired application.

Therefore, the compilation of appropriate data depends on weave/knit or non-woven structure and will be examined based on the following information:
Woven fabrics could have weave patterns defined as denim, twill, oxford, satin, gabardine, jacquard, gauze, cut pile, chiffon/georgette/shantung, and rib or basket weave to name a few. Knits could either be circular or flat and non- wovens could be bonded by thermalbond, spunlace, airlaid or needlepunch applications.

Upon analyzing the data, fine gauge needle selections will be determined with which to deconstruct the fabric into yarn sections prior to fiber refinement. For example, a needle used to deconstruct denim fabric will be substantially different than one used to deconstruct fine silk or a cable knit sweater. To underscore, it is significant to realize that the present invention specifically determines the manner in which incoming waste fabrics will be managed and processed.

Again, textile fabric waste should no longer be lumped together and viewed indiscriminately as simply scrap for recycling with the goal of maximizing the value of the raw materials for our industrial community. For example, an analysis of fabrications should conclude that the same needle used for cotton should not be used on wool; likewise, the needle used on wool would not be used on nylon, etc. Ironically, that very approach has been the modus operandi of textile waste recyclers and the basis for their technology throughout the centuries. Thus all of their products were developed from "shoddy fibers" and, in some cases, were a blend of high levels of virgin fibers and shoddy. This type of manufacturing has been propagated in niche plants under the guise of producing "environmentally friendly" items using lower efficiency fibers which have never been able to meet "big box" consumer demands of quality or on- time performance. Moreover, the production profile of these manufacturers has been relegated to low quality products whose functions have typically been acoustical, insulative or simply padding. To reiterate, all waste should be not treated equally to maximize the value of the raw materials for our industrial community.

The gradual deconstruction process of the present invention requires fabric pieces to be consolidated into a Feed Hopper to build up enough volume to create a mat as low as one but as high as three inches inches X.05 meters, or up to six meters wide, with an ideal width of 3 meters, as it moves onto a conveyor. The material is then fed through rubber grip rollers that hold the fabric in place while two rotary drums equipped with specialized needles and cooled by water, air or refrigerant, are used to penetrate the fabric and begin to deconstruct or slice it into small yarn sections. This action is predicated on the types of fabrics being deconstructed, whether woven or knit and their thread count, or non-woven. The cylinders containing these specialized needles rotate counter clockwise to maximize the amount of yarn sections that are able to be separated. A crucial issue in the successful process of this embodiment is that of material temperature control. Thus, the drums in the Fabric Deconstruction Group are consistently monitored for temperature and cooled by air, water or refrigerant relative to the nature of the raw material. High heat has been proven to denigrate fiber quality, yet in all other recycling and fiber regeneration embodiments, while heat is occasionally addressed and larger cylinders with steel teeth are built, they are still done so with metal "pins and lags" which are running at high velocity for increased productivity and are never temperature controlled. In the case of cotton, it begins to degrade and/or break at a temperature of 248°F relative to the heat element alone, its mechanical manipulation notwithstanding. Polypropylene begins to melt at a temperature of 150°F and if a fabric contains a low melt polyester, it can have a melt point of 110°F or less. Therefore, the rotating drums must maintain an average temperature of 98°F.

The cylinders are cooled in order to reduce heat buildup during rotation, thus keeping the surfaces cool so as not to tear, break, or otherwise damage any of the materials during the process. It cannot be overstated that in order to achieve a usable fiber in a high level application, strict attention must be paid its level of exposure to high temperature in the process. Gauges are installed on the cylinders to monitor and control their external temperatures and assure the correct adjustment in order to keep fiber stress to a minimum. While focusing on quality rejuvenated fibers it is important to understand that heat sensitivity is important in dealing with all types of fibers. Cotton fibers are sensitive to heat and will therefore break and create shorter fibers and/or dust in the process. Allowing excessive heat while separating cotton fabric into yarn pieces would be self-defeating in creating a quality end product. Should high temperatures be a factor in the rejuvenation of polypropylene fibers, polyester fibers or other heat sensitive fibers, they would fuse and result in poor performance and quality characteristics, along with production inefficiencies in downstream manufacturing, and hence in their particular consumer products.

The resultant yarn and fabric pieces will then be transferred into Catalyzed Vapor Chamber No. 1 which will add catalyzed moisture to the material, thus strengthening the overall fiber for continuous deconstruction and pre-fiberization. In the initial Catalyzed Vapor Chamber, a catalyzed vapor is added to soften the fabric and relax the twist so that it begins to untwist naturally without fraying. Relative to the desired fiber application, the types of vapor employed in the Catalyzed Vapor Chamber could be, for example, a cellulase enzyme, a surfactant or a silicone treatment. The yarns will then be transferred by conveyor into a second section containing two cylinders with removable wooden racks of needles which rotate counter clockwise and are focused on the further deconstruction of the fabric pieces which had not been untwisted into yarn elements in the prior operation. Once again, this cylinder remains relatively cool and temperature controlled.

As the fabric is being deconstructed into yarn segments, it is placed back onto the mat and conveyed into Catalyzed Vapor Chamber No. 2. It is at this stage that an organic agent is injected into Catalyzed Vapor Chamber No. 2 in order to strengthen the yarn elements for further processing. The type of catalyzed vapor used here, for example, might be a surfactant in the case of deconstructing a cotton fabric. If rejuvenating a natural hair fabric like cashmere, a complex catalyzed vapor that includes a poly (vinylamine-vinylformamide) copolymer with a carrier which would improve the strength of fibers that might have been weakened as a result of mechanical stress would be the preferred option.

If the situation called for polyester, nylon or polypropylene fiber to be deconstructed, a catalyzed vapor agent might be used in the eve that would allow the fabric to carry less static through the remainder of the process.

During each stage, individual soft yarns are moved out of the process to the final stage prior to fiber refinement and the heavier fabric pieces are moved forward through the mechanical process until they have been deconstructed into soft yarn elements. If there are elements of fabric that are not properly separated into yarn segments during each stage of the process, they are re-deposited two stages back in the process, for example by means of monitoring the weight of the output of each stage of the process and removing the fabric pieces with heavier weight meaning that they are not yet deconstructed into yarn segments. If sufficient yarn separation has not occurred in the fourth stage of the process, the elements of fabric would be re-deposited into Catalyzed Vapor Chamber No. 2. Should the separation be inadequate in the third stage of the process, the elements of fabric would be re-deposited into the initial fiber feeder prior to the mat development so as to prevent any unnecessary fiber breakage after the fabric has been deconstructed. The mechanical process to untwist the fibers becomes more aggressive at this stage, thus it is important to strengthen the fibers so there is no degradation. The success of this technology depends on the maintenance of the length and the strength of the fibers at each stage of the process and this is best achieved by having a Stage Gate Process of mechanical, chemical and vapor processing. To emphasize, when working with cotton or other natural fibers, the addition of warm moisture penetrates the fabric and increases the strength of the fiber to ensure that it is not being weakened and creating additional breakage during the process.

A mat of yarn segments coming out of Catalyzed Vapor Chamber No. 2 is positioned once again on a conveyor. The opened yarn moves into the enclosed chamber where a third set of dual rotary drums which are equipped with industrial needles and turning counter clockwise will similarly be used to further promote the deconstruction of the fabrics. As the yarn is being exposed, the fabric that has not been deconstructed is being reconfigured into a mat and conveyed to Catalyzed Vapor Chamber No. 3 to further relax and promote continuous deconstruction from its yarn state in the fabric.

The material then proceeds down the conveyor through a chamber to a semi-final drum covered with knife-like needles which will continue to delicately deconstruct the fabric pieces. The soft yarns are finally taken through Catalyzed Vapor Chamber No. 4, then through a final dual drum covered with knife-like needles which will finalize the deconstruction of the fabric pieces into soft yarn prior to Fiber Refinement. There are a number of catalyzed vapors that can be used based on downstream process requirements. However, if the downstream fibers are to be used in a medical or pharmaceutical grade product, an anti-microbial application or a biocide could begin to be administered at this stage.

It should be noted that the Catalyzed Vapor Chambers serve different purposes for each section and each type of fabric that is being presented. For example, cotton requires the addition of moisture along with a softener to its fiber at this stage. A catalyzed silicone vapor could be one of the many selections used for cotton in this case such as Softycon's SHP-C, Sofytcon's TRN or Rexamine CP 9194 AL. Other choices could be cellulase enzymes, surfactants or other silicone softening treatments. The ideal addition of moisture throughout the process of deconstruction will be 7% to 20% with the average being approximately 14%. If cotton is ever allowed to become dry, hot or brittle in the process of deconstruction the fibers will break. If the fibers break or become frayed during deconstruction their quality will be compromised downstream. Therefore, throughout the deconstruction and refining process it is important to add humidity to the material/yarn/fibers and maintain temperature control in order to eliminate short fibers and dust, which comes from dry or mechanically abused fiber. In the case of rayon fabrics, the catalyzed softener could be a blend of a non-ionic softener such as alkyl polyethanoxyether or polyoxyethylene alkyl ether, however, the application of water is not as critical here.

Products which have proven to be successful as part of the catalyzed vapor technology for these fabrics have been Perrustol CCF, Perrustol CCA or Softycon's RWT. The temperature of the Catalytic Vapor within the units can range from 100°C to 200°C, dependent on the application. At this stage in the production of synthetics such as polyester, polypropylene or nylon, cool mist vapor is utilized with the softening agent incorporating wick or ultrasonic technology to reduce the static electricity associated with these fabrics.

Having now conditioned the materials to a soft yarn state, it is necessary to individualize the fibers by taking them through the final stage in order to use them as a replacement for virgin fibers with no loss of efficiency in production speed or quality. The fiber refinement process developed in this technology takes the soft yarn segments and places them into individual fibers. Additionally, fibers are segregated at this point into different lengths with longer going into high count yarns, medium length fibers into medium count yarns and high speed non-wovens, and the shorter of these fibers going into course count yarns and lower speed non-wovens. The dust, or shorter fibers less than ½", are secured for wet laid processing. The fiber refinement process further removes neps (fiber bundles) that are created through handling during processing.

The refinement process begins by taking the unrefined fiber from the final stage of Fabric Deconstruction/pre-fiberization where it is layered and staged in a large blending box, which is important to the harmonious blending of the fiber for downstream processing. This procedure creates fiber parallelization resulting in quality equal, or superior, to that of virgin in both the actual fiber and its processability. A critical factor in the successful processing of these types of fibers is to ensure that they are never blown from successive procedures, but are rather transported by suction in order to reduce neps. Fibers are sent to a Feed Hopper where they are contained until the production line requires the sequential release of material. In prior art, textile-carding equipment is commonly utilized. However, it has been proven that the use of traditional textile carding equipment is not ideal for individual fiberization. Traditional carding equipment is primarily built to clean virgin cotton or other plant based fibers which contain dirt and trash by utilizing heavy gauged steel teeth or "lickerins," etc. and then create a "card sliver," which is then spun into yarn. While the final refined fiber resulting from this technology will be made available to many downstream products such as quality textile yarn, the use of this type of carding equipment to refine fibers is not preferable within this method's protocol. In our experience, the use of heavy gauged teeth creates additional breakage which results in a loss of critical fiber that is important to the overall product mix. We do not find this technology to be successful in creating consistent quality fiber for downstream applications. The equipment described in those embodiments could be used to create yarn in textile mills, but not to create a consistent quality rejuvenated textile fiber.

A new approach to Fiber Refinement became necessary when years of process research and production led to the conclusion that while fiber of adequate quality was indeed being created using traditional carding machinery, fiber breakage remained a source of frustration. This was discovered when making application for US/12/605,341. Carding is created to clean cotton that has significant trash, leaves, and stems coming from the cotton fields. The equipment in itself created neps, short fiber and even if short fiber wasn't created it weakened the fiber overall. Others have continued to use carding alone as their only source of rejuvenation of fiber. Fiber fibrillization constituted a negative outcome and the significant loss of good fiber was problematic since the equipment itself was originally employed to remove trash, stems, leaves and other debris from fibrous materials in the early stages of carding. While the use of traditional carding equipment was good for organic fibers such as cotton in the spinning process, it was inadequate for rejuvenating fiber. It became necessary to develop a solid solution for cotton, polyester, polypropylene fibers, silk, bamboo or other specialty fibers that are all plentiful in the rejuvenation marketplace and need attention to quality. Therefore, we resolutely abandoned a portion of our previous art and forged ahead in researching a solution to the dilemma of refining high-level fibers that would flourish in quality products and maintain their efficiency throughout downstream processing.

As the new process begins and fibers are properly untwisted from their yarn state and appropriately integrated to yield as much randomness in the blend of materials as possible, it becomes time to individualize the fibers and bale them for downstream processing. The fibers are moved from the blending area by conveyor into the system pass automated metal detection, and then conveyed through the fiber conditioner during the initial stage of the process. With some fibers this step serves to eliminate static, especially in the case of blended fibers such as polyester/cotton or perhaps polyester/rayon. In some of the rejuvenated fibers such as cotton, this step strengthens and increases moisture that may have been lost in the system. It is also important in the case of cotton to add a conditioner to help reduce the neps that can occur with mechanical handling. With fibers such as PLA or Bamboo, it has been important to add an agent to allow it to "glide" through the refinement process. There are many processing aids that are used and these are examples of those associated with a few types of fibers. The utilization of a unique processing aid is predicated on each fiber type that has been identified in the list of fibers that can be rejuvenated. The fiber is then delivered onto a gauged wire main cylinder by a gauged wire feeding roll where it is combed much like hair is combed, first from the top and then underneath. As the fibers continue to their specific series of finely gauged wired rolls, it is important to understand the difference in the wire function and each type of roll necessary to refine the unique types of fiber being delivered. A cotton wire will be gauged much differently than a polyester synthetic wire, and wire that will refine a jute or raffia fiber will be more unique than a wire that will refine a bamboo fiber. Therefore, each refining unit has distinct individual settings for speed and wired rolls.

These rolls comb the fibers both from the top and underneath, similar to strokes employed in the brushing of human hair. This process will parallelize and actually stretch the fibers to their optimum length as well as remove any shorter fibers that may have formed and move them to the appropriate areas for downstream production.

The fibers are then evacuated from the Fiber Refining Units by a vacuum system into their appropriate balers. Considering the meticulous care with which the raw materials, as well as each fiber and yarn segment, are being handled throughout the overall process, there remain three different lengths of fiber that will be naturally produced during rejuvenation. The categorization of those fibers constitute short, which are 6mm or less, and both medium and long, whose qualification is dependent upon the types of raw materials being deconstructed. These fibers are captured throughout the system, but especially in the Fiber Refinement Section and are baled to be used in downstream products accordingly. This dynamic is part of the value system of rejuvenation. If only 70% of the fibers can be utilized, then the cost of rejuvenation is 30% higher for the fiber that is being used. When all the fiber can be used, the cost of rejuvenation can be spread across 100% of the production costs and can create a 100% sustainable product.

The fibers will subsequently be delivered to the next stage of combing where they will be combed on both top and underneath to further individualize them. The final step and one of the critical steps of the purification of the fiber process is that a very thin web of fiber is exposed to the UVC Chamber where the entire fiber web is exposed for complete purification and disinfection of the fibers. Lastly, the fibers will be suctioned from the wire pneumatically, using a negative pressure system and sent to a baler having passed through final metal detection in preparation for their downstream processing.

In certain circumstances, it may be necessary to blend other fibers with the fabric pieces, soft yarns and/or fiber tufts obtained above to produce target product. In such cases, ratio of the other fibers blended with the fabric pieces, soft yarns and/or fiber tufts is between 2/98 and 99/1. These rejuvenated fibrous materials may also be converted to a composite material.

The rejuvenated fibrous material obtained above may be subject to various further processing, examples of which may include the following: The rejuvenated fibrous material may be spun into threads or yarns, and the threads or yarns may then be weaved into woven fabrics, or knitted into fabrics, rope or cable, cord, trim, fringe, or braids.

The rejuvenated fibrous material may also be converted into spunlaced nonwoven fabric, needlepunch nonwoven fabric, thermalbond nonwoven fabric, carded bond nonwoven fabric, spunbond nonwoven fabric or airlaid nonwoven fabric, and such fabric may then be further converted into personal care, cosmetic, baby, medical, filtration, geotextile or other industrial products.

According to the above disclosure, a person skilled in the art may make suitable modifications and changes to the above embodiments. Therefore, the present invention is not limited by the above disclosure and the embodiment described. Modifications and changes to the present invention should fall within the scope of the present invention as defined by the claims. Besides, although certain technical terms have been used throughout the specification, the technical terms are intended for ease of explanation and are not intended to restrict the present invention in any ways.

## Claims

1. A method of processing waste fabrics to rejuvenated fibrous materials, comprising the steps of:
obtaining fabric data for each bale of incoming waste fabrics and storing the fabric data in a database;
obtaining target data relating to target product requirements;
processing the fabric data stored in the database and the target data according to predetermined algorithms to generate a rejuvenation processing recipe which specifies bales information relating to bales of incoming waste fabrics selected for further rejuvenation processing, and rejuvenation processes information relating to a series of processes and corresponding process parameters for each of the series of processes for processing the selected bales of incoming waste fabrics to obtain rejuvenated fibrous materials specific to the target product requirements;
selecting, according to the bales information of the rejuvenation processing recipe, corresponding bales of incoming waste fabrics for further rejuvenation processing;
subjecting the selected bales of incoming waste fabrics to processes specified by the rejuvenation processes information of the rejuvenation processing recipe to obtain rejuvenated fibrous materials specific to the target product requirements.

2. The method of processing waste fabrics to rejuvenated fibrous materials as in Claim 1, wherein the processes specified by the rejuvenation processes information of the rejuvenation processing recipe comprise:
(e1) opening and simultaneously blending the selected bales of incoming waste fabrics to obtain initially opened and blended fabric pieces;
(e2) cleaning the initially opened and blended fabric pieces to obtain cleaned fabric pieces;
(e3) subjecting the cleaned fabric pieces to gradual deconstructing process to obtain soft yarns or fiber tufts;
(e4) blending the soft yarns or fiber tufts;
(e5) subjecting the blended soft yarns or fiber tufts to fiber conditioning and refinement for parallelization to obtain refined fibers;
(e6) extracting the refined fibers of all lengths for final baling of fibers.

3. The method of processing waste fabrics to rejuvenated fibrous materials as in Claim 1, wherein the fabric data in Step (a) includes composition of the incoming waste fabric, approximate yarn count of the incoming waste fabric, types of treatments or finishes that the incoming waste fabric have received during production of the incoming waste fabric, and color and shade of the incoming waste fabric.

4. The method of processing waste fabrics to rejuvenated fibrous materials as in Claim 1, wherein the bales of incoming waste fabrics contain fibers including one or a blend of the following: natural fibers from plant or vegetable, animal hairs, metallic fibers, fibers transformed from natural polymers, synthetic fibers, inorganic fibers.

5. The method of processing waste fabrics to rejuvenated fibrous materials as in Claim 4, wherein the natural fibers from plant or vegetable include cotton, kapok, kupro, flax, linen, hemp, jute, ramie, kenaff, straw, banan, pina, papyrus, alfagras, fique, alginate, urena, nettle, broom, apocynum, raffia, bamboo, sisal, abaca, henequen, phormium, rosella, acacia, aloe, yucca, coconut, and elastodiene.

6. The method of processing waste fabrics to rejuvenated fibrous materials as in Claim 4, wherein the animal hairs include wool, camel, vicuna, alpaca, llama, mohair, cashmere, horse hair, goat hair, rabbit hair, yak, beaver, chitosan, silk, chiengora, and qiviut.

7. The method of processing waste fabrics to rejuvenated fibrous materials as in Claim 4, wherein the metallic fibers include gold, silver, aluminized yarns or aluminized plastic yarns.

8. The method of processing waste fabrics to rejuvenated fibrous materials as in Claim 4, wherein the fibers transformed from natural polymers include viscose, bamboo (regenerated), modal, lyocell, acetate, triacetate, milk protein, PLA, and byssus.

9. The method of processing waste fabrics to rejuvenated fibrous materials as in Claim 4, wherein the synthetic fibers include polyamides, polyesters, polyurethanes, polyvinyls, polyolefins, polypropylene, copolymers, elastomeric fibers, modacrylics, aramid, paramids, and PBI.

10. The method of processing waste fabrics to rejuvenated fibrous materials as in Claim 4, wherein the inorganic fibers include as glass fiber, silicic acid glass, carbon, ceramic, steel, inox, copper and basalt.

11. The method of processing waste fabrics to rejuvenated fibrous materials as in Claim 1, wherein the incoming waste fabrics are post-industrial waste fabrics which are sorted preliminarily by fabric color and fabric type.

12. The method of processing waste fabrics to rejuvenated fibrous materials as in Claim 1, wherein Step (a) comprises conducting laboratory tests to the incoming waste fabrics; the laboratory tests include one or more of the following: burn test, solubility test, dye staining test, Fourier Transform Infrared spectroscopy, chemical reaction test, burn rate test, elemental analysis, color and shade test, UV spectroscopy imaging and detection.

13. The method of processing waste fabrics to rejuvenated fibrous materials as in Claim 1, wherein after Step (a) the bales of incoming waste fabrics are sorted into batches according to the obtained fabric data.

14. The method of processing waste fabrics to rejuvenated fibrous materials as in Claim 2, wherein before Step (e2), the initially opened and blended fabric pieces are reduced to an appropriate size for conforming to downstream production equipment, and thereafter blended to avoid segregation and to unify composition of the fabric pieces.

15. An apparatus using UV-A and UV-C to treat textile materials, comprising:
a segregation chamber having an input end and an output end,
one or more conveyor belts for traversing incoming textile materials from the input end to the output end,
a source of UV-A light disposed within the segregation chamber, capable of emitting radiation in the wavelength of between about 315 nanometers and about 400 nanometers, such that incoming textile materials exposed to the UV-A light will fluoresce if they include synthetic fibers and/or have been treated with optical brighteners,
wherein the source of UV-A light is of a sufficient wavelength and intensity, and is disposed at a suitable distance from the conveyor belts, such that textile materials comprising synthetic fibers and/or which have been treated with optical brighteners will fluoresce,
optionally, a vacuum or an air jet, which vacuum or air jet is sized so as to be capable of separating textile materials which include synthetic fibers and/or have been treated with optical brighteners from textile materials formed from natural fibers which have not been treated with optical brighteners; and
a sterilization chamber which receives textile materials output either directly or indirectly from the segregation chamber; wherein textile materials output received indirectly from the segregation chamber can comprise one or more of fibers, yarns, woven materials, or non-woven materials, wherein
a UV-C radiation source is disposed within the sterilization chamber, which UV-C radiation source irradiates the sterilization chamber with UV-C radiation at a sufficient wavelength and at a sufficient intensity to disinfect or sterilize the fibers, yarns, non-woven, or woven textile materials that traverse through the sterilization chamber, and/or to modify the surface of the textile materials to increase wettability, absorbability or reduce pilling of the textile materials.
